# EUROPEAN PATENT SPECIFICATION

(11) **EP 4 037 757 B1**
(45) Date of publication and mention of the grant of the patent: **27.08.2025**
(21) Application number: 20872394.0
(22) Date of filing: 01.10.2020
(51) Int. Cl.: A61N 1/08, A61N 1/05

(54) **LEAD FOR AN ACTIVE IMPLANTABLE MEDICAL DEVICE**
LEITUNG FÜR EIN AKTIVES IMPLANTIERBARES MEDIZINPRODUKT
SONDE POUR DISPOSITIF MÉDICAL IMPLANTABLE ACTIF

(30) Priority: 04.10.2019 AU 2019903747
(43) Date of publication of application: 10.08.2022
(73) Proprietor: Saluda Medical Pty Ltd, Artarmon, New South Wales 2064 (AU)
(72) Inventor: HARTUNG, Dirk, Artarmon, New South Wales 2064 (AU)
(74) Representative: Abel & Imray LLP
(86) International application number: PCT/AU2020/051054
(87) International publication number: WO 2021/062480

(56) References cited:
- US-A1- 2008 058 902
- US-A1- 2008 058 902
- US-A1- 2012 041 528
- US-A1- 2013 123 600
- US-A1- 2013 245 734
- US-A1- 2014 188 201
- US-A1- 2015 025 611
- US-A1- 2015 099 958
- US-A1- 2017 095 667
- US-A1- 2018 185 650

## Description

### Cross-Reference to Related Applications

The present application claims priority from Australian Provisional Patent Application No 2019903747 filed on 4 October 2019.

### Technical Field

The present disclosure relates to lead for an active implantable medical device (AIMD) for implanting into tissue of a patient.

### Background

Medical devices having one or more active implantable components, generally referred to herein as active implantable medical devices (AIMDs), have provided a wide range of therapeutic benefits to patients over recent decades. AIMDs often include an implantable, hermetically sealed electronics module, and a device that interfaces with a patient's tissue, sometimes referred to as a tissue interface. The tissue interface may include, for example, one or more instruments, apparatus, sensors or other functional components that are permanently or temporarily implanted in a patient. The tissue interface is used to, for example, diagnose, monitor, and/or treat a disease or injury, or to modify a patient's anatomy or physiological process.

In particular applications, an AIMD tissue interface includes one or more conductive electrical contacts, referred to as electrodes, which deliver electrical stimulation signals to, or receive signals from, a patient's tissue. The electrodes are typically disposed in a biocompatible electrically non-conductive member, and are electrically connected to the electronics module. The electrodes and the non-conductive member are collectively referred to herein as an electrode assembly.

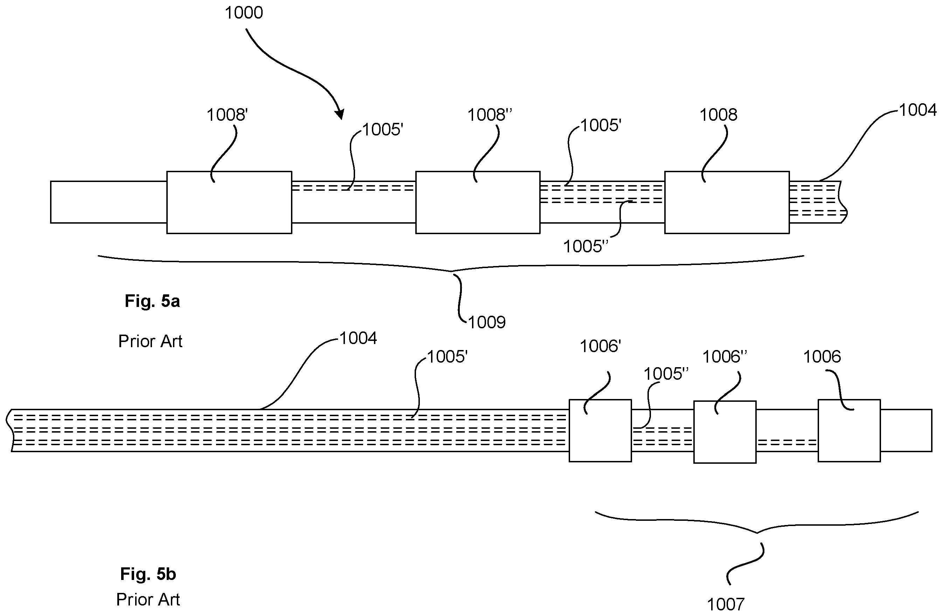

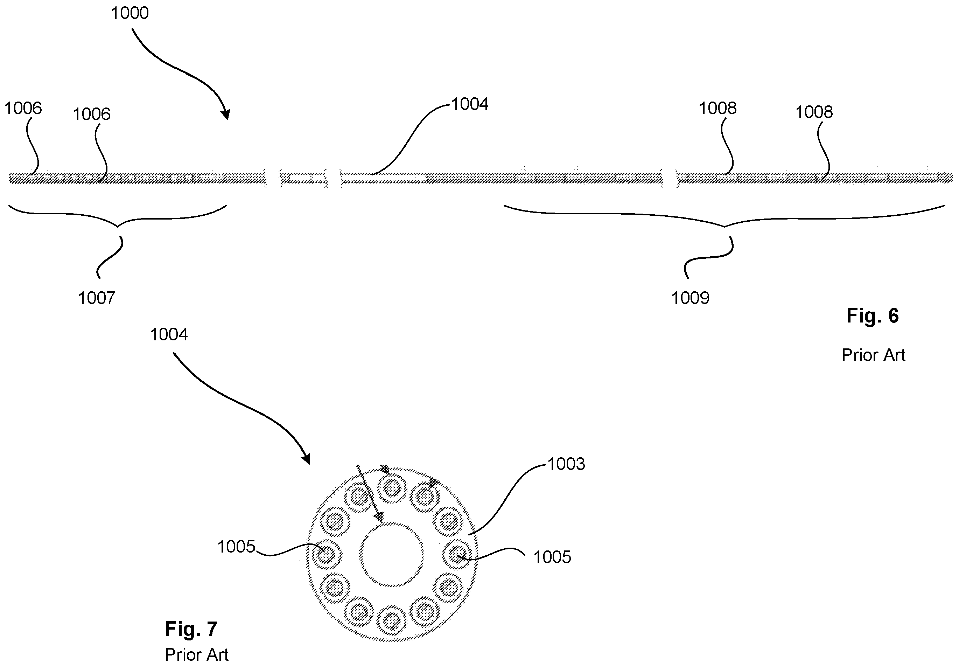

For neuro-stimulators, the tissue interface is a stimulating lead 1000 which delivers electrical pulse to a specific nerve or tissue. This lead 1000 may consist of a long thin non-conductive (and insulating) body 1004 and a number of conductive rings 1006, 1008 at both ends 1007, 1009 of the body 1004. Referring to Figs. 5a, 5b, 6 and 7, the rings 1008 at a therapeutic end 1009 are known as electrodes and the rings 1006 at the connector end 1007 are known as contacts, where the electrodes are connected to the contacts along the long thin non-conductive body 1004. An example of the long thin non-conductive body 1004 is shown in the cross-section in Fig. 7 that shows conductive wires 1005 surrounded by a non-conductive body 1003. These conductive wires 1005 are sized to have lengths to conduct signals between corresponding conductive rings, such as the length of conductive wire 1005' that spans between 1006' and 1008' in Figs. 5a and 5b. A different length of conductive wire 1005" spans between conductive rings 1006" and 1008".

Patients with an implanted neuro-stimulator and associated lead may have issues undergoing magnetic resonance imaging (MRI). The MRI uses three types of fields to create an image: a static magnetic field; a radiofrequency (RF) magnetic field; and a gradient magnetic field. Exposure to these fields may cause heating to the leads. This heating may result in tissue burns and damage (which may not be immediately felt by the patient). Another potentially damaging effect is damage to the implant due to radiofrequency energy being transmitted from the lead. This can lead to reprogramming, damage to the implant or explant of the implant. Additionally, the MRI could cause a temporary unintended stimulation due to induced voltage through the assembly and system. US2008/0058902 discloses a medical device including a resonance tuning module located in the housing of the medical device, connected to a lead. The resonance tuning module further includes a control circuit, the control circuit acting to determining a resonant frequency for the device, and an adjustable impedance circuit to change the combined resonant frequency of the medical device and lead. US2015/0025611 discloses an electrical implantable lead, with a elongated body including a plurality of lumens. At least one non-linear lumen extends longitudinally along a portion of the lead body and includes a plurality of crests and troughs.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

### Summary

There have been attempts to provide designs for MRI safe leads. US patent publication US 8798767 B2 illustrates a method of reducing the heat caused by MRI conditions. This document suggests coiling conductors in a multi-layer structure, with each coil layer electrically connected to the next to provide parallel conductive paths. However, this method may result in high inductance when exposed to MRI radiation. US patent publication US 9050457 B2 uses a similar approach with a lead body and multi-layer coil conductor within the length of the lead body. The stiffness of the multi-layer coil conductor is similar to the lead body, ensuring consistent mechanical properties of the lead. US patent publication US 9302101 B2 uses a different approach with the lead body providing an additional path for containing conductive material. This path spans at least a section of the length of the lead for conducting the induced RF energy away from the conductive wire of the lead.

In light of the above mentioned issues, it would be advantageous to have an electrode assembly, such as one used in an implantable medical device, that may be implanted in a patient whilst the patient is undergoing magnetic resonance imaging. This may include providing an implantable electrode assembly which, when exposed to an MRI environment, does not generate significant heat in the leads due to electromagnetic currents. In may be further advantageous for an implantable medical device that can operate during magnetic resonance imaging without, or with reduced, side effects described above.

The present invention is defined by the appended claims.

There is disclosed a lead for an active implantable medical device comprising: an elongated, biocompatible, electrically non-conductive body having a centre section between a first portion at a proximal end and a body extension at a distal end; a plurality of electrical connectors at the first portion; a plurality of electrodes at a second portion of the elongated body, wherein the second portion is between the centre section and the body extension ; and a plurality of electrically conductive filaments inside the elongated body to connect the electrical connectors to corresponding electrodes, wherein each of the plurality of electrically conductive filaments include corresponding filament extension sections in the body extension that extends towards the distal end beyond a most distal electrode of the plurality of electrodes at the second portion.

In some examples, a length of each of the filament extension sections is 10mm or more. In yet further examples, the length of each of the filament extension sections is in the range of 10mm to 50mm.

In some examples, the lead further comprises a biocompatible, electrically non-conductive seal at the body extension to insulate the filament extension sections.

In some examples of the lead, each of the plurality of conductive filaments have a common physical and/or electrically equivalent overall length.

In further examples, each electrical distance between each electrical connector and corresponding electrode is shorter that the common overall length.

In some examples of the lead, each of the plurality of electrical connectors are located separately along a first length of the first portion.

In some examples of the lead, each of the plurality of electrodes are located separately along a length of the second portion.

In some examples of the lead, the electrical connectors and/or electrodes are ring shaped.

In some examples, the lead further comprises a central lumen to receive a stylet.

There is also provided a method of manufacturing a lead for an active implantable medical device, comprising: forming a multi-lumen elongated biocompatible, electrically non-conductive body, the elongated body having a centre section between a first portion at a proximal end and a body extension at a distal end; locating a plurality of electrically conductive filaments through the lumens of the elongated body, wherein the plurality of electrically conductive filaments include corresponding filament extension sections that extend into the body extension beyond a most distal electrode of the plurality of electrodes at the second portion; forming a plurality of electrical connectors at the first portion, wherein each of the electrical connectors are electrically connected to respective electrically conductive filaments; forming a plurality of electrodes at a second portion of the elongated body, wherein the second portion is between the centre section and the body extension, and the plurality of electrodes are connected, via corresponding electrically conductive filaments, to corresponding electrical connectors.

In some examples, the method further comprises: sizing each of the plurality of electrically conductive filaments to a common overall length.

In some examples, the elongated body is formed by extruding the biocompatible, electrically non-conductive material with the multi-lumens.

In some examples, the method further comprises sealing an end of the body extension to insulate the filament extension sections.

### Brief Description of Drawings

Figs. 1a and 1b illustrate an example of a lead for an active implantable medical device, whereby Fig. 1a shows a distal end and Fig. 1b shows a proximal end;
Fig. 2a is a perspective view of a distal end of the lead including a body extension with corresponding filament extension sections;
Fig. 2b illustrates a cross-section of the lead at the proximal end and through an electrical connector;
Figs. 3a and 3b are test results showing, respectively, the magnitude of complex electric fields at a distal end with and without the body extension;
Fig. 4 is a flow diagram of a method of manufacturing a lead for an active implantable medical device;
Fig. 5a and 5b illustrate an example of a prior art lead;
Figs. 6 illustrates another view of a prior art lead; and
Fig.7 illustrates a cross section of the prior art lead in Fig. 6.

### Description of Embodiments

It will be appreciated by persons skilled in the art that numerous variations and/or modifications may be made to the above-described embodiments, without departing from the scope of the present invention which is defined by the appended claims.

Aspects of the present disclosure are generally directed to an electrode assembly for an active implantable medical device (AIMD). An AIMD may include an implantable electronics module and a tissue interface. The electrode assembly that, at least in part, forms the tissue interface.

The electrode assembly may be used with one type of AIMD, a neuro stimulator, and more specifically a deep brain stimulator or spinal cord stimulator. Deep brain stimulators are a particular type of AIMD that deliver electrical stimulation to a patient's brain, while spinal cord stimulators deliver electrical stimulation to a patient's spinal column. As used herein, deep brain stimulators and spinal cord stimulators refer to devices that deliver electrical stimulation alone or in combination with other types of stimulation. It should be appreciated that embodiments of the present disclosure may be implemented in any brain stimulator (deep brain stimulators, cortical stimulators, etc.), spinal cord stimulator or other neuro stimulator now known or later developed, such as cardiac pacemakers/defibrillators, functional electrical stimulators (FES), pain stimulators, etc. Embodiments of the present disclosure may also be implemented in AIMDs that are implanted for a relatively short period of time to address acute conditions, as well in AIMDs that are implanted for a relatively long period of time to address chronic conditions.

The electrode assembly in accordance with embodiments of the present disclosure are not limited to devices that deliver electrical stimulation signals to a patient. For instance, in certain embodiments, the electrode assembly may be used to receive, record or monitor the physiological response of a patient's tissue to, for example, a therapy. In such embodiments, the electrodes receive a signal from the patient's tissue representing the physiological response. An electrode assembly of the present disclosure that delivers electrical stimulation signals to, or receives signals from, a patient's tissue may also include one or more other components, such as therapeutic agent delivery systems, sensors, etc., that interface with the patient's tissue.

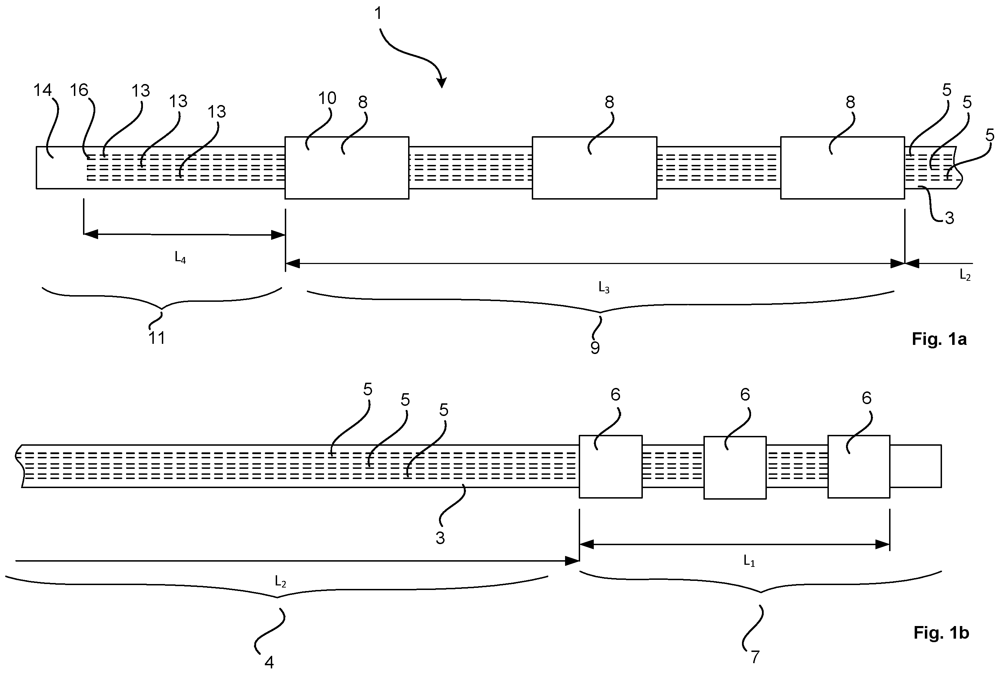

An example of a lead 1 for an active implantable medical device 99 is illustrated in Figs. 1a and 1b (which show opposite ends of the same lead 1). The lead 1 includes an elongated, biocompatible, electrically non-conductive body 3. The elongated body 3 extends from a first portion 7, to a centre section 4, to a second portion 9, and finally to a body extension 11.

A plurality of electrical connectors 6 are at the first portion 7 near a proximal end whereby the connectors 6 are used to electrically connect to the active implantable medical device 99. A plurality of electrodes 8 are at the second portion 9 near a distal end of the elongated body 3, such that the electrodes 8 are between the centre section 4 and the body extension 11. The electrodes 8 deliver therapy to the patient.

A plurality of electrically conductive filaments 5 (represented by broken lines) are located inside the elongated body 3 to connect the electrical connectors 6 to corresponding electrodes 8. Furthermore, the plurality of electrically conductive filaments 5 include corresponding filament extension sections 13 in the body extension 11.

The filament extension sections 13 in the body extension 11 moves the reflection point 16 of the electromagnetic waves along the elongated body 3 away from the most distal electrode 10 at the second portion 9. Such electromagnetic waves are excited along the lead 1 during MRI scans.

An example of the specific components of the lead 1 will now be described in detail.

### The first portion 7

The first portion 7, including the plurality of connectors 6, are configured to be inserted into the AIMD 99, whereby the connectors 6 are in electrical connection with respective connectors inside the AIMD. In one example, the connectors 6 are configured to be received in medical grade connector/contact systems such as those under the trade name "Bal Conn" offered by "Bal Seal Engineering". The connectors 6 are substantially annular (i.e. ring-shaped) and typically constructed of a biocompatible and electrically conductive material. The annular construction permits good electrical contact with the receiving contact in the AIMD. Suitable material for the connectors 6 may include, but is not limited to, platinum, iridium, and/or alloys thereof. In other examples, the connectors 6 can include a core of high conductance material such as cobalt, chromium, molybdenum or alloys thereof with a coating of metals such as platinum, tantalum, niobium, titanium or alloys thereof.

The connectors 6 are located separately along axis length L₁ of the first portion 7. In Fig. 1b, this includes three connectors 6 spaced along the first portion 7 for illustrative purposes. However, it is to be appreciated that additional connectors 6, for additional channels, can be used. In some examples, this can include up to a dozen or more channels (with a corresponding dozen connectors 6, electrically conductive filaments 5, and electrodes 8). The whole length L1 of the first portion 7 is typically inserted into the AIMD.

In addition to the connectors 6, the first portion 7 includes non-conductive part(s) that support the connectors 6. In some examples, this includes the same material, and can be part of, the elongated, biocompatible, electrically non-conductive body 3.

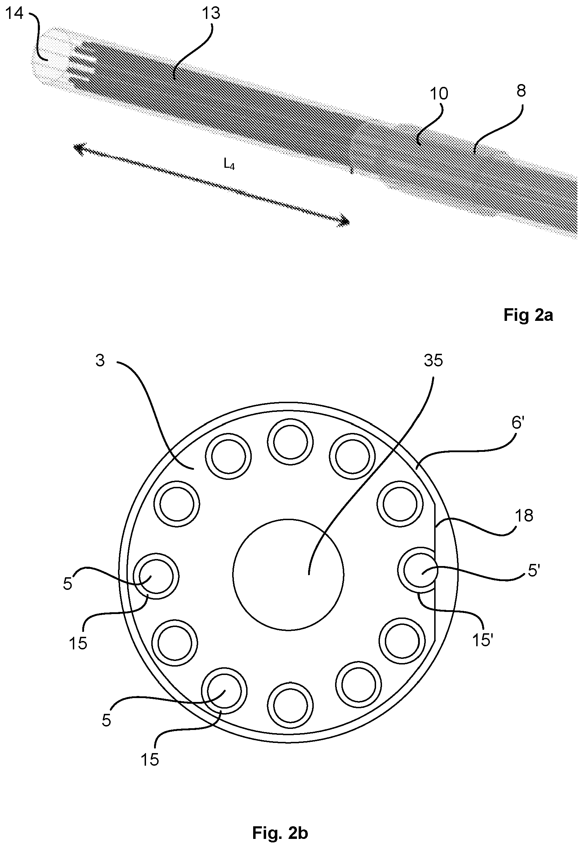

A particular example will now be described with reference to Fig. 2b which is a cross-section of the first portion 7 through one of the connectors 6, of a twelve channel lead 1. This includes the biocompatible, electrically non-conductive body 3 with a central lumen 35, and a dozen outer lumens 15. The central lumen 35 is provided to receive a stylet to aid insertion and implantation of the lead 1 after which the stylet is withdrawn. The outer lumens 15 house the electrically conductive filaments 5, which in this case includes a dozen electrically conductive filaments 5.

A particular example of electrically connecting the connector 6' to an electrically conductive filament 5' will now be described with reference to Fig. 2b. Part of the electrically non-conductive body 3 is thinned 18 or removed to expose a particular outer lumen 15'. This allows an electrically conductive filament 15'inside the outer lumen 15' to be in electrical contact with the electrical conductor 6' located around the periphery of the body 3. It is to be appreciated that the order of assembling these components can be varied.

In one example, the electrically conductive filaments 5 are inserted into the outer lumens 15 (such as by a drawn filled tubing process) and the connectors 6 are subsequently attached to the first portion 7 of the body 3. In other examples, the connectors 6 are positioned on the body 3 before the electrically conductive filaments 5 are inserted in the outer lumens 15.

In the above example, the body 3 and electrical conductive filaments 5 extend into, and are part of, the first portion 7. In alternative examples, separate material or components can form the first portion 7. This can include a separate electrical conductive filament in the first portion 7 that is electrically connected to the electrically conductive filament 5 in the centre portion 4. Similarly, a body of the first portion 7 can be separately made, but connected to, the centre portion 4 of the body 3.

### The body 3 and centre section 4

The centre section 4 of the body 3 includes the biocompatible, electrically non-conductive body 3 and the electrically conductive filaments 5 in the outer lumens 15. In examples where a stylet is used, the centre section 4 also has a central lumen 35 to receive the stylet.

The centre section 4 has a length L₂, which is typically the longest portion of the elongated body 4. In typical examples, the centre section 4 encloses the electrically conductive filaments 5 to minimise conduction between the electrically conductive filaments 5 to tissue immediately surrounding the centre section 4.

The body 3 is made of biocompatible, electrically non-conductive material that can include thermoplastic polyurethanes (TPUs) such as those under the trade name "pellethane" offered by "The Lubrizol Corporation". In some examples, the body 3 is made from an extrusion of a flexible material with multiple lumens. In some examples, the body 3 and the electrically conductive filaments 5 are mated together with a drawn filled tubing process.

The electrically conductive filaments 5 is preferably selected from a configuration of biocompatible materials. This can include single core or multi strand wires. In another example, this can include a composite of a medical grade alloy, with the trade name "35N LT" offered by Fort Wayne Metals, having a silver core. In another specific example, the conductive filaments 5 include a wire jacket of 35N LT (Nickel-Cobalt-Chromium alloy) per ASTM F562 and of a composition which includes ≤ 0.01wt% titanium, and a wire core of silver 28% of the cross-sectional area of the electrically conductive filament 5.

### The second portion 9

The second portion 9 includes a plurality of electrodes 8, 10 to deliver therapy to the patient. In some examples, the number of electrodes 8 are the same as the number of corresponding connectors 6, and in turn, the same number of electrically conductive filaments 5 that form the electrical connection.

The size and spacing of the electrodes 8 located along length L₃ is selected based on the anticipated therapy to the patient. Typically, each electrode 8 has a longer axial length than the corresponding connector 6.

The electrodes 8, in some examples, can be assembled to the lead 1 in a similar way to the connectors 6 in the first portion 7. Thus in some examples, the structure shown in Fig. 2b similarly reflects the structure in the second portion 9. Furthermore, the electrodes 8 can be made of the same, or a similar, biocompatible electrically conductive material.

The non-conductive part(s) of the second portion 9 can be integrally formed with the body 3 of the centre section 4 and the first portion 7. Similarly, the electrically conductive filaments 5 are integrally formed and extend from the centre section 4 into the second portion 7. The electrically conductive filaments 5 continue through the second portion 9 and to the body extension 11 discussed below.

It is to be appreciated that in alternative examples, the electrically conductive filaments 5 in the second portion 9 can be separately formed, but electrically connected, to the electrically conductive filaments 5 in the centre section 4. Similarly the non-conductive part(s) of the second portion may, in an alternative example, be separately formed but connected to the centre section 4.

### The body extension 11

Referring to Figs. 1a and 2a, the body extension 11 is at the distal end of the lead 1 and adjacent the second portion 9. The purpose of the body extension 11 is to extend the electrically conductive filaments 5, by a Length L₄ of the filament extension section 13, past the most distal electrode 10 of the plurality of electrodes 8. The Length L₄ moves the reflection point 16 of an electromagnetic wave propagating along the lead 1 away from the most distal electrode 10.

In some examples, the body extension 11 is formed as part of the biocompatible, electrically non-conductive body 3 that makes up the first portion 7, centre section 4, and second portion 9. This can include an extrusion of flexible material with a multi-lumens, whereby the end is sealed with an electrically non-conductive seal 14. The seal 14 insulates (to an extent) the filament extension section 13 from the immediate surrounding tissue.

In some examples, this seal 14 can be a plug of material injected into the lumens 15, 35. In other examples, the end of the body extension 11 can be dipped into a sealing material. In yet another examples, the seal 14 is formed by closing the lumens 15, 35 in the electrically non-conductive body 3 material by welding, such as heat, ultrasonic welding, resistance welding, laser welding, etc. A similar seal may be provided at the first portion to close off the ends of the outer lumens 15 at the proximal end.

### Configurations of the body extension 11

In some examples, the filament extension section 13 has a Length L₄ of 10 mm or more. In some examples, the Length L₄ is in the range of 10mm to 50mm (inclusive).

In some examples, each of the electrically conductive filaments 5 are sized to have a common overall length. In some examples, this common overall length is a physical overall length. In other examples, the common overall length is an electrically equivalent overall length. In yet further examples, the electrically conductive filaments share both common physical and electrically equivalent overall length. The common overall length may assist in minimising the generation of differential electromagnetic modes and induced differential voltages propagating between the filaments 5 in the body 3.

In some examples, the common overall length of the electrically conductive filament 5 is the sum of L₁, L₂, L₃, and L₄.

In some examples, the electrical distance between each electrical connector 6 and the corresponding electrode 8 is shorter than the common overall length. This difference in length is typically at least Length L₄ of the filament extension section 13.

The leads 1 are selected from a length suitable for the therapy and patient characteristics. In some examples, the leads 1 are in the range of 600mm to 900mm in length. In yet other examples, the lead are in the range of 500mm to 1200mm in length.

### Test data

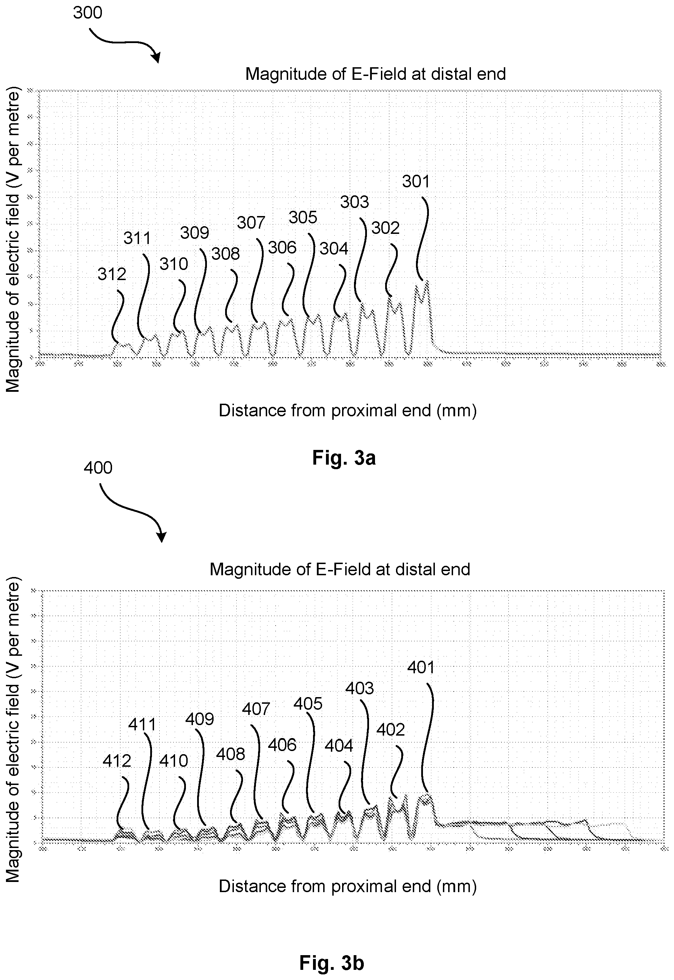

Figs. 3a and 3b illustrate complex electric field magnitudes at a distal end (i.e. body near the second portion 9) for a 600mm long lead 1 with a dozen of electrodes 9 (and corresponding dozen of electrically conductive filaments 5 and electrical connectors 6). The diagram 300 in Fig. 3a illustrates the magnitude of complex electric fields at the distal end without a body extension 11. In particular, the highest magnitude corresponds to the first electrode 301 located at the most distal portion (i.e. around 600mm from the proximal end), with a magnitude of approximately14 V/m. The other electrodes 302, 303, 304, 305, 306, 307, 308, 309, 310, 311, 312 have a smaller magnitude, with the twelfth electrode 312 (closer to the proximal end) having the smallest magnitude.

The diagram 400 in Fig. 3b illustrates the magnitude of complex electric field at the distal end with a body extension 11 ranging from 10 mm to 50 mm. These different lengths are represented by the different lines in the diagram. The highest magnitude corresponds to the first electrode 401 located at the most distal portion (i.e. around 600mm from the proximal end), with a magnitude of approximately 9.5 V/m. The other electrodes 402, 403, 404, 405, 406, 407, 408, 409, 410, 411, 412 have a smaller magnitude, with the twelfth electrode 412 (closer to the proximal end) having the smallest magnitude

In both scenarios (i.e. with and without the body extension 11), a plane electromagnetic wave with an axiolateral electric field of 1V/m at 128 MHz was used to excite the distal end. The leads and AIMD (in the form of an implanted pulse generator) were embedded in a gelled saline solution to simulate tissue. In this test, the lead 1 with the body extension 11 at the distal end a decrease in complex electric field magnitude by more than 30%.

### Fabrication

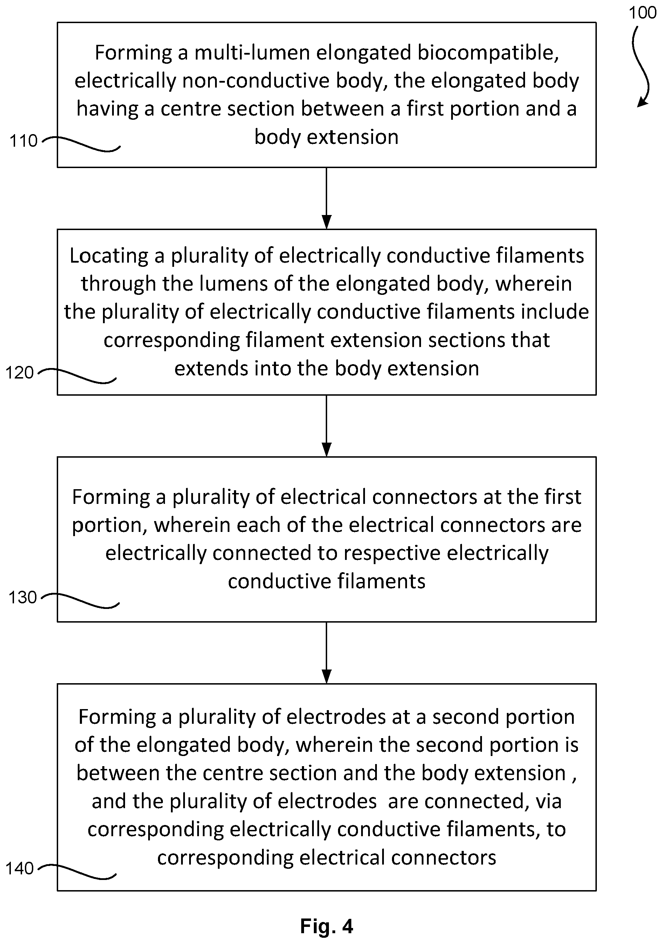

Referring to Fig. 4, there is also disclosed a method of manufacturing a lead 1 for an active implantable medical device 99. This includes forming 110 a multi-lumen 15 elongated biocompatible, electrically non-conductive body 3, wherein the elongated body 3 has a centre section 4 between a first portion 7 and a body extension 11. In some examples, this includes extruding the elongated body 3 with multiple lumens. The method also includes locating 120 a plurality of electrically conductive filaments 5 through the lumens 15 of the elongated body 3, wherein the plurality of electrically conductive filaments 5 include corresponding filament extension sections 13 that extend into the body extension 11. As noted above, some examples of include locating and securing the electrically conductive filaments 5 using a drawn filled tubing technique. The method further includes forming 130 a plurality of electrical connectors 6 at the first portion 7, wherein each of the electrical connectors 6 are electrically connected to respective electrically conductive filaments 5. The method 100 also includes forming 140 a plurality of electrodes 8 at a second portion 9 of the elongated body 3, wherein the second portion 9 is between the centre section 4 and the body extension 11, and the plurality of electrodes 8 are connected, via corresponding electrically conductive filaments 5, to corresponding electrical connectors 6.

In some examples, it may be possible to perform some of these steps in other sequences. For example, the step of forming the electrodes 140 can precede the step of forming the connectors 130.

The method 100 may also include additional steps. For example, this can include steps of sealing one or more ends of the lead and or sizing the length of the electrically conductive filaments 5.

It will be appreciated by persons skilled in the art that numerous variations and/or modifications may be made to the above-described embodiments, without departing from the scope of the present invention which is defined by the appended claims.

## Claims

1. A lead (1) for an active implantable medical device (99) comprising:
- an elongated, biocompatible, electrically non-conductive body (3) having a centre section (4) between a first portion (7) at a proximal end and a body extension (11) at a distal end;
- a plurality of electrical connectors (6) at the first portion (7);
- a plurality of electrodes (8) at a second portion (9) of the elongated body (3),
wherein the second portion (9) is between the centre section (4) and the body extension (11); and
- a plurality of electrically conductive filaments (5) inside the elongated body (3) to connect the electrical connectors (6) to corresponding electrodes (8),
- wherein each of the plurality of electrically conductive filaments (5) include corresponding filament extension sections (13) in the body extension (11) that extends towards the distal end beyond a most distal electrode (10) of the plurality of electrodes (8) at the second portion (9).

2. A lead (1) according to claim 1 wherein a length (L₄) of each of the filament extension sections (13) is 10mm or more.

3. A lead (1) according to claim 1 wherein a length (L₄) of each of the filament extension sections (13) is in the range of 10mm to 50mm.

4. A lead (1) according to any one of the preceding claims further comprising a biocompatible, electrically non-conductive seal (14) at the body extension (11) to insulate the filament extension sections (13).

5. A lead (1) according to any one of the preceding claims wherein each of the plurality of conductive filaments (5) have a common physical and/or electrically equivalent overall length.

6. A lead (1) according to claim 5 wherein each electrical distance between each electrical connector (6) and corresponding electrode (8) is shorter that the common overall length.

7. A lead (1) according to any one of the preceding claims, wherein each of the plurality of electrical connectors (6) are located separately along a first length (L₁) of the first portion (7).

8. A lead (1) according to any one of the preceding claims, wherein each of the plurality of electrodes (8) are located separately along a length (L₃) of the second portion (9).

9. A lead (1) according to any one of the preceding claims wherein electrical connectors (6) and/or electrodes (8) are ring shaped.

10. A lead (1) according to any one of the preceding claim further comprising a central lumen (35) to receive a stylet.

11. A method (100) of manufacturing a lead (1) for an active implantable medical device (99), comprising:
- forming a multi-lumen (15) elongated biocompatible, electrically non-conductive body (3), the elongated body having a centre section (4) between a first portion (7) at a proximal end and a body extension (11) at a distal end;
- locating a plurality of electrically conductive filaments (5) through the lumens (15) of the elongated body (3), wherein the plurality of electrically conductive filaments (5) include corresponding filament extension sections (13) that extend towards the distal end and into the body extension (11) beyond a most distal electrode (10) of the plurality of electrodes (8) at the second portion (9);
- forming a plurality of electrical connectors (6) at the first portion (7), wherein each of the electrical connectors (6) are electrically connected to respective electrically conductive filaments (5);
- forming a plurality of electrodes (8) at a second portion (9) of the elongated body (3), wherein the second portion (9) is between the centre section (4) and the body extension (11), and the plurality of electrodes (8) are connected, via corresponding electrically conductive filaments (5), to corresponding electrical connectors (6).

12. A method (100) according to claim 11 further comprising:
- sizing each of the plurality of electrically conductive filaments (5) to a common overall length.

13. A method (100) according to any one of claims 11 to 12 wherein the elongated body (3) is formed by extruding the biocompatible, electrically non-conductive material with the multi-lumens (15).

14. A method (100) according to any one of claims 11 to 13 further comprising sealing an end of the body extension (11) to insulate the filament extension sections (13).

## Patentansprüche

1. Leitung (1) für eine aktive implantierbare medizinische Vorrichtung (99), aufweisend:
- einen länglichen, biokompatiblen, elektrisch nicht leitfähigen Körper (3) mit einem Mittelabschnitt (4) zwischen einem ersten Bereich (7) an einem proximalen Ende und einer Körperverlängerung (11) an einem distalen Ende;
- eine Mehrzahl von elektrischen Verbindern (6) an dem ersten Bereich (7);
- eine Mehrzahl von Elektroden (8) an einem zweiten Bereich (9) des länglichen Körpers (3), wobei sich der zweite Bereich (9) zwischen dem Mittelabschnitt (4) und der Körperverlängerung (11) befindet; und
- eine Mehrzahl von elektrisch leitfähigen Filamenten (5) im Inneren des länglichen Körpers (3), um die elektrischen Verbinder (6) mit entsprechenden Elektroden (8) zu verbinden,
- wobei die mehreren elektrisch leitfähigen Filamente (5) jeweils entsprechende Filamentverlängerungsabschnitte (13) in der Körperverlängerung (11) aufweisen, die sich in Richtung auf das distale Ende über eine distalste Elektrode (10) der Mehrzahl von Elektroden (8) an dem zweiten Bereich (9) hinaus erstrecken.

2. Leitung (1) nach Anspruch 1,
wobei eine Länge (L₄) jedes der Filamentverlängerungsabschnitte (13) 10 mm oder mehr beträgt.

3. Leitung (1) nach Anspruch 1,
wobei eine Länge (L₄) jedes der Filamentverlängerungsabschnitte (13) im Bereich von 10 mm bis 50 mm liegt.

4. Leitung (1) nach einem der vorhergehenden Ansprüche,
die außerdem eine biokompatible, elektrisch nicht leitfähige Dichtung (14) an der Körperverlängerung (11) aufweist, um die Filamentverlängerungsabschnitte (13) zu isolieren.

5. Leitung (1) nach einem der vorhergehenden Ansprüche,
wobei alle der Mehrzahl von leitfähigen Filamenten (5) eine gemeinsame physikalische und/oder elektrisch äquivalente Gesamtlänge aufweisen.

6. Leitung (1) nach Anspruch 5,
wobei jeder elektrische Abstand zwischen jedem elektrischen Verbinder (6) und der entsprechenden Elektrode (8) kürzer ist als die gemeinsame Gesamtlänge.

7. Leitung (1) nach einem der vorhergehenden Ansprüche,
wobei jeder der Mehrzahl von elektrischen Verbindern (6) separat entlang einer ersten Länge (L₁) des ersten Bereichs (7) angeordnet ist.

8. Leitung (1) nach einem der vorhergehenden Ansprüche,
wobei jede der Mehrzahl von Elektroden (8) separat entlang einer Länge (L₃) des zweiten Bereichs (9) angeordnet ist.

9. Leitung (1) nach einem der vorhergehenden Ansprüche,
wobei die elektrischen Verbinder (6) und/oder Elektroden (8) ringförmig sind.

10. Leitung (1) nach einem der vorhergehenden Ansprüche,
die außerdem ein zentrales Lumen (35) zur Aufnahme eines Mandrins aufweist.

11. Verfahren (100) zum Herstellen einer Leitung (1) für eine aktive implantierbare medizinische Vorrichtung (99), das folgende Schritte aufweist:
- Bilden eines mehrlumigen (15), länglichen, biokompatiblen, elektrisch nicht leitfähigen Körpers (3), wobei der längliche Körper einen Mittelabschnitt (4) zwischen einem ersten Bereich (7) an einem proximalen Ende und einer Körperverlängerung (11) an einem distalen Ende aufweist;
- Anordnen einer Mehrzahl von elektrisch leitfähigen Filamenten (5) durch die Lumen (15) des länglichen Körpers (3), wobei die Mehrzahl von elektrisch leitfähigen Filamenten (5) entsprechende Filamentverlängerungsabschnitte (13) aufweist, die sich in Richtung auf das distale Ende und in die Körperverlängerung (11) hinein über eine distalste Elektrode (10) der Mehrzahl von Elektroden (8) an dem zweiten Bereich (9) hinaus erstrecken;
- Bilden einer Mehrzahl von elektrischen Verbindern (6) an dem ersten Bereich (7), wobei jeder der elektrischen Verbinder (6) mit jeweiligen elektrisch leitfähigen Filamenten (5) elektrisch verbunden wird;
- Bilden einer Mehrzahl von Elektroden (8) an einem zweiten Bereich (9) des länglichen Körpers (3), wobei sich der zweite Bereich (9) zwischen dem Mittelabschnitt (4) und der Körperverlängerung (11) befindet und die Mehrzahl von Elektroden (8) über entsprechende elektrisch leitfähige Filamente (5) mit entsprechenden elektrischen Anschlüssen (6) verbunden werden.

12. Verfahren (100) nach Anspruch 11,
das weiterhin aufweist:
- Bemessen jedes der Mehrzahl von elektrisch leitfähigen Filamenten (5) auf eine gemeinsame Gesamtlänge.

13. Verfahren (100) nach einem der Ansprüche 11 bis 12,
wobei der längliche Körper (3) durch Extrudieren des biokompatiblen, elektrisch nicht leitfähigen Materials mit den mehreren Lumen (15) gebildet wird.

14. Verfahren (100) nach einem der Ansprüche 11 bis 13,
das ferner das Abdichten eines Endes der Körperverlängerung (11) umfasst, um die Filamentverlängerungsabschnitte (13) zu isolieren.

## Revendications

1. Fil (1) pour un dispositif médical implantable actif (99) comprenant :
- un corps allongé, biocompatible, électriquement non conducteur (3) présentant une section centrale (4) entre une première partie (7) au niveau d'une extrémité proximale et une extension de corps (11) au niveau d'une extrémité distale ;
- une pluralité de connecteurs électriques (6) au niveau de la première partie (7) ;
- une pluralité d'électrodes (8) au niveau d'une seconde partie (9) du corps allongé (3), dans lequel la seconde partie (9) se trouve entre la section centrale (4) et l'extension de corps (11) ; et
- une pluralité de filaments électriquement conducteurs (5) à l'intérieur du corps allongé (3) pour connecter les connecteurs électriques (6) aux électrodes correspondantes (8),
- dans lequel chacun de la pluralité de filaments électriquement conducteurs (5) comprend des sections d'extension de filament correspondantes (13) dans l'extension de corps (11) qui s'étend vers l'extrémité distale au-delà d'une électrode la plus distale (10) de la pluralité d'électrodes (8) au niveau de la seconde partie (9).

2. Fil (1) selon la revendication 1, dans lequel une longueur (L₄) de chacune des sections d'extension de filament (13) est de 10 mm ou plus.

3. Fil (1) selon la revendication 1, dans lequel une longueur (L₄) de chacune des sections d'extension de filament (13) est dans la plage de 10 mm à 50 mm.

4. Fil (1) selon l'une quelconque des revendications précédentes, comprenant en outre un joint biocompatible et électriquement non conducteur (14) au niveau de l'extension de corps (11) pour isoler les sections d'extension de filament (13).

5. Fil (1) selon l'une quelconque des revendications précédentes, dans lequel chacun de la pluralité de filaments conducteurs (5) présente une longueur totale physique et/ou électriquement équivalente commune.

6. Fil (1) selon la revendication 5, dans lequel chaque distance électrique entre chaque connecteur électrique (6) et l'électrode correspondante (8) est plus courte que la longueur totale commune.

7. Fil (1) selon l'une quelconque des revendications précédentes, dans lequel la pluralité de connecteurs électriques (6) sont localisés chacun séparément le long d'une première longueur (L₁) de la première partie (7).

8. Fil (1) selon l'une quelconque des revendications précédentes, dans lequel la pluralité d'électrodes (8) sont localisées chacune séparément le long d'une longueur (L₃) de la deuxième partie (9).

9. Fil (1) selon l'une quelconque des revendications précédentes, dans lequel les connecteurs électriques (6) et/ou les électrodes (8) sont en forme d'anneau.

10. Fil (1) selon l'une quelconque des revendications précédentes, comprenant en outre une lumière centrale (35) pour recevoir un stylet.

11. Procédé (100) de fabrication d'un fil (1) pour un dispositif médical implantable actif (99), comprenant les étapes consistant à :
- former un corps allongé biocompatible et électriquement non conducteur (3) à lumières multiples (15), le corps allongé présentant une section centrale (4) entre une première partie (7) au niveau d'une extrémité proximale et une extension de corps (11) au niveau d'une extrémité distale ;
- localiser une pluralité de filaments électriquement conducteurs (5) à travers les lumières (15) du corps allongé (3), dans lequel la pluralité de filaments électriquement conducteurs (5) comprend des sections d'extension de filament correspondantes (13) qui s'étendent vers l'extrémité distale et jusque dans l'extension de corps (11) au-delà d'une électrode la plus distale (10) de la pluralité d'électrodes (8) au niveau de la seconde partie (9) ;
- former une pluralité de connecteurs électriques (6) au niveau de la première partie (7), dans lequel les connecteurs électriques (6) sont connectés chacun électriquement connecté à des filaments électriquement conducteurs respectifs (5) ;
- former une pluralité d'électrodes (8) au niveau de la seconde portion (9) du corps allongé (3), dans lequel la seconde portion (9) est située entre la section centrale (4) et l'extension de corps (11), et la pluralité d'électrodes (8) sont connectées, via une pluralité de filaments électriquement conducteurs (5), pour correspondre à une pluralité de connecteurs électriques (6).

12. Procédé (100) selon la revendication 11, comprenant en outre l'étape consistant à :
- dimensionner chacun de la pluralité de filaments électriquement conducteurs (5) à une longueur totale commune.

13. Procédé (100) selon l'une quelconque des revendications 11 à 12, dans lequel le corps allongé (3) est formé en extrudant le matériau biocompatible, électriquement non conducteur avec les lumières multiples (15).

14. Procédé (100) selon l'une quelconque des revendications 11 à 13, comprenant en outre l'étape consistant à sceller une extrémité de l'extension de corps (11) pour isoler les sections d'extension de filament (13).
